# EUROPEAN PATENT APPLICATION

(11) **EP 0 853 943 A1**
(43) Date of publication of application: **22.07.1998**
(21) Application number: 97114701.2
(22) Date of filing: 25.08.1997
(51) Int. Cl.: A61K 35/78, A61K 7/48

(54) **Antiallergic agent**

(30) Priority: 19.12.1996 JP 339887/96
(71) Applicant: SUNTORY LIMITED, Kita-ku, Osaka-shi, Osaka (JP)
(72) Inventor: Uehara, Masami, Otsu-shi, Shiga-ken (JP); Sugiura, Hisashi, Otsu-shi, Shiga-ken (JP); Fujii, Wataru, Ibaraki-shi, Osaka (JP); Yang, Zhi-bo, Otsu-shi, Shiga-ken (JP); Suwa, Yoshihide, Ibaraki-shi, Osaka (JP)
(74) Representative: Hansen, Bernd, Dr. Dipl.-Chem.

(57) **Abstract**

The antiallergic agent, antiinflammatory agent, anti-atopic dermatitis agent or antipsoriatic agent of the present invention is efficacious in preventing, reducing or relieving symptoms due to inflammation or allergic reactions, and in particular, it is useful in preventing, reducing or relieving the symptoms of atopic dermatitis and psoriasis.
The agent of the present invention, which contains oolong tea extract as the active ingredient, is excellent in safety and exerts no side-effect even over continuous and prolonged administration.

## Description

This invention relates to an agent containing oolong tea extract as the active ingredient. More particularly, it relates to an agent having antiinflammatory, antiallergic, anti-atopic dermatitis or antipsoriatic effects and foods, beverages and cosmetics containing the same.

Various epidemiological surveys indicate that allergic diseases continue to increase in recent years. In particular, cedar pollen hypersensitivity and atopic dermatitis have been on the remarkable increase and thus even brought about a social problem. It is estimated that the increase in these allergic diseases is caused by not only increase in allergens but also changes in our environment such as air pollution, use of food additives and changes in eating habits. It is the fundamental method for treating allergic diseases to eliminate or not take the causative allergens. It has been a practice to administer drugs appropriately selected depending on the severity of the symptoms or the onset mechanism of the disease.

Allergic reactions are classified into four groups, i.e., the types I to IV depending on the causative immunoglobulins and cells participating therein. The types I to III allergies are immunological reactions in which humoral antibodies participate. They are called immediate-type allergies, since allergic reactions appear quickly therein. On the other hand, the type IV allergy is a cell-mediated immunological reaction in which not any antibody but sensitized lymphocytes participate. It is also called delayed-type allergy.

Diseases typified by allergic rhinitis, bronchial asthma and urticaria fall within the category of the type I allergy. Allergic reaction of the type I is a supernumerary biological reaction in which a chemical mediator such as histamine or leukotriene is released from mast cells or basophils via IgE antibody to thereby induce vasodilation, elevation of vascular permeability, constriction of bronchial smooth muscle, stimulation of nerve terminus, etc. Accordingly, it is a common practice for treating the I type allergic diseases to use antihistaminic agents together with antiallergic agents having an effect of inhibiting the release of chemical mediators from mast cells. However, antihistaminic agents and basic antiallergic agents frequently cause side-effects such as sleepiness, thirstiness or gastrointestinal disorders, which results in a problem in safety in association with the continuous administration thereof over a long time.

The type IV allergic reaction is a delayed-type reaction in which T cells participate. Namely, antigenic information is transferred via antigen presenting cells (Langerhans' cells, macrophages, etc.) to sensitized T cells which subsequently release various cytokines, thus inducing delayed-type inflammatory reaction due to the accumulation of eosinophils or macrophages. Allergic contact dermatitis is a typical example of the diseases which occur depending on the type IV allergic reaction. Fundamental methods for treating these allergic diseases of the type IV include the external steroid therapy and detailed guide to life style. Steroids act on T cells and macrophages and suppress the production of cytokines to thereby exert a specific therapeutic effect on eczema. When used in a large dose or over a long time, however, steroids cause a problem in safety, namely, the frequent occurrence of serious side-effects including systemic ones such as hypoadrenocorticism and topical ones such as dermatrophia, flushing and capillarectasia. Although antihistaminic agents and antiallergic agents are supplementarily employed therewith, these drugs can establish only limited antipruritic effects.

As an example of allergic skin diseases, atopic dermatitis may be cited and the number of patients suffering from this disease has been on the remarkable increase year by year. There have been proposed various factors causing atopic dermatitis for a long time. On the basis of the pathological findings and clinical characteristics of atopic dermatitis, there is a tendency in recent years to support the theory that this disease is associated with allergic reaction of not the type I but the type IV. This theory is grounded in the presence of atopic dermatitis patients lacking allergic reaction of the type I. It is also suggested that atopic dermatitis is associated with allergic reactions of both the types I and IV. Atopic dermatitis is a hereditary disease and its cause has never been strictly specified so far. It has been immunologically clarified that psoriasis, which is one of allergic skin diseases typified by vulgar psoriasis, is also induced by dermal monocyte infiltration by T cells and macrophages. Many of pathological and histological findings of this diseases are equivalent to those of atopic dermatitis. The fundamental practice for treating atopic dermatitis, which is a hereditary disease showing repeated worsening and amelioration, resides in controlling dermatitis. Similarly, psoriasis is frequently worsened and becomes chronic by external stimulus. Pustular psoriasis is a lesion occurring in those carrying predispositions for psoriasis such as past history or family history. In each case, it is the fundamental therapy to control the skin symptoms to thereby prevent worsening.

Tea is a world-wide popular beverage and has been taken by us over 2,000 years. Various effects of tea have been known from old times. Moreover, it has been recently clarified that tea extract has various physiological functions including antioxidant, antibacterial and blood cholesterol-regulating effects. Tea is roughly classified into 3 types, namely, non-fermented tea (green tea), semi-fermented tea (oolong tea) and fermented tea (black tea) depending on difference in production process, though these products are all made of leaves of a same plant (*Camellia sinensis*). The flavors of these products can be easily distinguished from each other by merely taking them in the usual manner, which indicates that the components contained in these extracts largely differ from each other owing to the differences in the production processes.

With respect to the relation between tea extract and allergy, JP(Kokai) Hei 3-258726 proposes an antiallergic agent, which contains black tea and oolong tea extract as the main ingredient, for treating the type I allergy with the use of the inhibitory effect on the release of histamine from mast cells as indication. It is further reported that oolong tea stem extract and catechins, i.e., the purification fraction thereof would suppress the passive cutaneous anaphylaxis (PCA) in rats which are model animals of the type I allergy (Biol. Pharm. Bull., Vol. 18, No. 5, pp 683 - 686, 1995). However, there has never been reported that oolong tea extract would suppress the type IV allergic reaction or relate thereto. It is also reported that green tea extract is positive in the evaluation of delayed type contact allergy in accordance with the CCET method with the use of guinea pigs (J. Cosmetics, Vol. 10, No. 2, pp 79 - 85, 1986). It is furthermore reported that green tea extract serves as an allergen.

With the disclosure of the pathologic physiology of allergic diseases, there have been developed remedies appropriate for the pathology thereof. However, the treatment with these drugs is frequently accompanied by the occurrence of side-effects. Moreover, these drugs can achieve only transient therapeutic effects and, therefore, fail to essentially ameliorate allergic diathesis. By considering the tendency to increase in allergic diseases due to changes in the contemporary life style and environment and the serious effects thereof on living body, it has been urgently required to develop antiallergic agents which are actually efficacious and safe over prolonged and continuous administration. From the viewpoint of preventive medicine, it has been required to establish a preventive treatment with the use of, for example, foods, beverages or cosmetics which can be taken or employed every day. Accordingly, it is an object of the present invention to provide remedies and preventives for allergic diseases, in particular, those for atopic dermatitis and vulgar psoriasis originating in natural materials, being free from any side-effect, showing a high safety even in continuous administration over a long time and being usable in foods, beverages or cosmetics.

To solve the above-mentioned problems, the present inventors have conducted extensive studies for screening an antiallergic substance with the use as indication of the inhibitory effect on auricular contact dermatitis of mice and the inhibitory effect on delayed food edema of mice (i.e., model animals of the type IV allergic reaction). As a result, they have confirmed that oolong tea extract has the desired effects. Further, the present inventors have attempted to administer the oolong tea extract to patients with atopic dermatitis the cause of which has never been specified. As a result, they have newly found that the oolong tea extract is highly efficacious against atopic dermatitis, thus completing the present invention.

Accordingly, the present invention provides an antiallergic agent or an antiinflammatory agent containing oolong tea extract as the active ingredient and aiming at preventing, reducing or relieving inflammatory or allergic symptoms, in particular, preventing and treating atopic dermatitis and psoriasis.

Oolong tea is a semi-fermented tea originating in China and cultivated mainly in Fujian, Guangdong and Taiwan. Since canned oolong tea was put on the market and its convenience earned popularity, oolong tea has been quickly spread in Japan where consumers prefer sugar-free beverages. So oolong tea is now widely taken in Japan. Because of being a tea beverage, it is taken in a large amount everyday for a long time. Nevertheless, no harmful effect of oolong tea has been found out so far. On the contrary, animal experiments with the use of rats and rabbits indicate that the continuous intake of oolong tea for a long time causes no problem in safety [Masaru Otsuru and Kimio Nishimura, "Uroncha no Seitai ni Ataeru Eikyo nitsuite (Effects of Oolong Tea on Living Body)", from "Kohi to Ocha no Genkyo to Tenbo (The Present State and Prospects of Coffee and Tea)", Aug. 31, 1989, Kogyo Gijutsu-kai]. That is to say, oolong tea to be used in the present invention is a highly safe beverage, and, therefore, can be given to human with a high safety. From the standpoint of patients, oolong tea, which is familiar as an everyday beverage, can be pleasantly and positively taken without any discomfort or anxiety. Thus oolong tea can be smoothly taken for preventive purposes too.

### [Fig. 1]

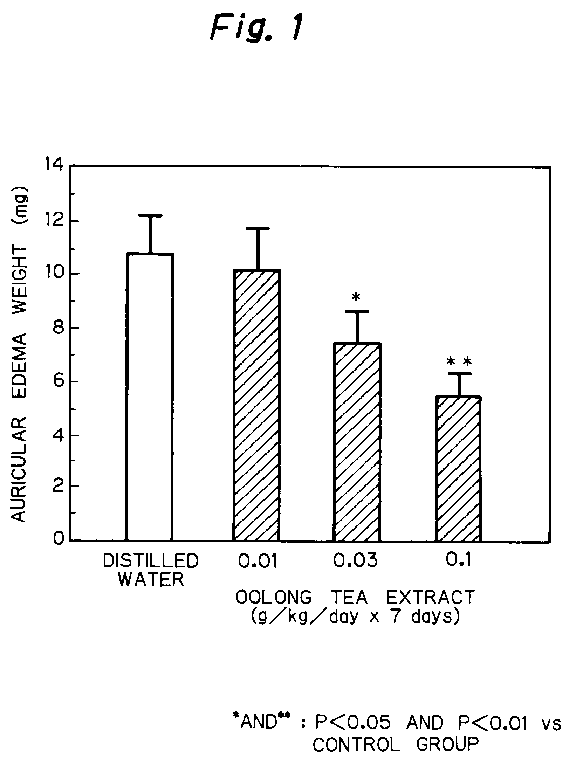

Fig. 1 is a graph showing the results of Test Example 1, i.e., the difference in inhibitory effect on mouse ear contact dermatitis between the test groups with the administration of 0.01, 0.03 and 0.1 g/kg/day of the oolong tea extract of the present invention and the control group with the administration of distilled water.

### [Fig. 2]

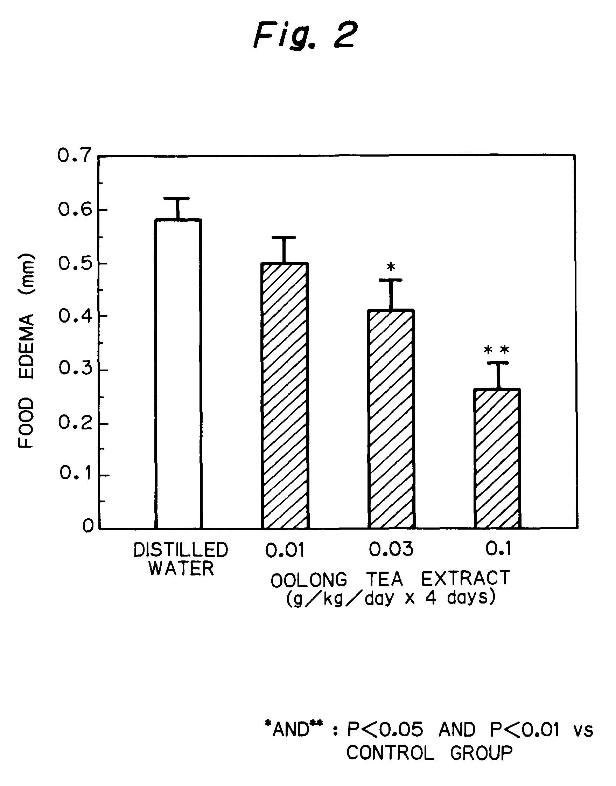

Fig. 2 is a graph showing the results of Test Example 2, i.e., the difference in inhibitory effect on mouse delayed-type foot edema between the test groups with the administration of 0.01, 0.03 and 0.1 g/kg/day of the oolong tea extract of the present invention and the control group with the administration of distilled water.

The oolong tea extract to be used in the present invention can be obtained by extracting oolong tea leaves with, for example, an aqueous solvent. The aqueous solvent to be used in the extraction may be either water alone or an arbitrary mixture of water with one or more polar solvents such as lower alcohols (methanol, ethanol, etc.) or acetone. When it is taken into consideration that the extract is finally added to drugs, foods or beverages and taken or employed in cosmetics, it is preferable from the viewpoint of safety to select water, ethanol or a mixture thereof among these solvents.

In the extraction, the ratio of the oolong tea leaves to the solvent is not particularly restricted. It is preferable to use the solvent in an amount 2 to 1,000 times by weight, still preferably 5 to 100 times by weight from the viewpoints of extraction performance and efficiency, as much as the oolong tea leaves.

Although the extraction temperature is not particularly restricted too, it is convenient to perform the extraction within a range from room temperature to the boiling point of the solvent, in particular, the boiling point of the solvent at room temperature under atmospheric pressure. The extraction time preferably ranges from 10 seconds to 24 hours.

It is sometimes observed that sodium hydrogen carbonate is added to the solvent by which oolong tea leaves are extracted or sodium hydrogen carbonate, sodium L-ascorbate, etc. are added to the extract to thereby improve its preference, etc. Since these additives exert no undesirable effect, the products thus obtained are also usable as the oolong tea extract in the present invention.

As the oolong tea extract of the present invention, it is preferable to use one having a concentration ranging from 0.1 to 40% by weight (Brix; the content of solid matters). Oolong tea usually taken as a beverage has a concentration of from 0.1 to 1% by weight. It is therefore preferable from the viewpoint of preference that the oolong tea extract has a concentration falling within the above range and can be drunk as such, in the case of oral administration. When the bitter taste is undesirable from the viewpoint of preference, it is possible to sweeten the oolong tea extract by adding sugars thereto. When the oolong tea extract has a concentration higher than the range usually employed as beverages, it sometimes tastes bitter when drunk as such. In this case, it may be taken preferably in the form of a thickened extract, a freeze-dried powder, etc. optionally processed into tablets, capsules, etc. When the oolong tea extract has a concentration lower than the range usually employed as beverages, it can be drunk as such. In this case, however, it is often undesirable from the viewpoint of preference. To achieve the desired concentration, the extract can be appropriately diluted with water or concentrated by, for example, evaporation before using. When the oolong tea extract is to be administered to young people, there sometimes arises the problem of caffeine hypersensitivity. In such a case, use can be made of the oolong tea extract from which caffeine has been already eliminated in a conventional manner, if necessary, without worsening the effects of the present invention. The same will apply to external preparations.

The oolong tea extract provided by the present invention can be orally administered either as such (i.e., in the form of the extract) or after diluting with water, etc. Alternatively, the extract may be formulated together with publicly known pharmaceutical carriers into preparations. For example, the extract may be formulated into liquid preparations for oral use such as syrups. Furthermore, it may be formulated into a thickened extract, a powder, etc. and blended with pharmaceutically acceptable carriers to thereby give solid preparations for oral use such as tablets, capsules, granules, dusts, etc. As the pharmaceutically acceptable carriers, use can be made of various organic and inorganic materials commonly employed in the art as carriers. For example, fillers, lubricants, binders, disintegrating agents, etc. may be used in solid preparations, while solvents, fillers, suspending agents, binders, etc. may be used in liquid preparations. Furthermore, use can be made of various additives such as preservatives, antioxidants, coloring agents and sweeteners, if necessary.

Appropriate examples of the fillers include lactose, sucrose, D-mannitol, starch, crystalline cellulose and light anhydrous silicic acid. Appropriate examples of the lubricants include magnesium stearate, calcium stearate, talc and colloidal silica. Appropriate examples of the binders include bound cellulose, sucrose, D-mannitol, dextrin, hydroxypropylcellulose, hydroxypropylmethylcellulose and polyvinyl pyrrolidone. Appropriate examples of the disintegrating agents include polyethylene glycol, propylene glycol, D-mannitol, benzyl benzoate, ethanol, trisaminomethane, cholesterol, triethanolamine, sodium carbonate and sodium citrate. Appropriate examples of the solvents include purified water, alcohols and propylene glycol. Appropriate examples of the suspending agents include ethanolamine stearate, sodium lauryl sulfate, laurylamino-propionic acid, lecithin, benzalkonium chloride, benzethonium chloride, surfactants such as glycerol monostearate, and hydrophilic polymers such as polyvinyl alcohol, polyvinyl pyrrolidone, sodium carboxymethylcellulose, methylcellulose, hydroxymethylcellulose, hydroxyethylcellulose and hydroxypropylcellulose. Appropriate examples of the preservatives include parahydroxybenzoates, chlorobutanol, benzyl alcohol, phenethyl alcohol, dehydroacetic acid and sorbic acid. Appropriate examples of the antioxidants include sulfites and ascorbic acid.

The oolong tea extract provided by the present invention can be administered as such (i.e., as the extract). Alternatively, it may be formulated into a thickened extract, powder, etc. which is then processed into foods or beverages. It may be blended with edible materials commonly employed and carriers acceptable in manufacturing foods and beverages and then served as foods or beverages. Examples of the beverages include oolong tea beverages, tea beverages prepared by mixing with other tea, carbonated beverages, fruit beverages, lactic acid beverages, sport beverages and soya milk. Examples of the confectionery include biscuits, chocolates, candies, chewing gums, snacks, fried cakes, fresh cakes, Japanese cakes, ice creams and jellies. Examples of the foods include breads, noodles, processed soybean products such as *tofu* (bean curd), dairy products such as yoghurt and butter, processed meat products such as ham and sausage, processed egg products such as *tamago-yaki* (Japanese omelet) and *chawan-mushi* (custard-like dish steamed in a cup), processed marine products such as *tsukuda-ni* (small fishes and shellfishes boiled in sweetened soy sauce), ground fish meat products such as *kamaboko* (boiled fish paste), seasonings such as sauce, dressing, mayonnaise and *furikake* (rice topping) and prepared dishes such as curry, stew, hamburg steak and soup. These products can be each produced in a conventional manner.

Examples of the carriers acceptable in manufacturing foods and beverages include sweeteners such as sucrose, glucose, fructose, isomerized liquid sugars, fructoligosaccharide, aspartame, sorbitol and stevia; coloring agents such as red cabbage colorant, grape pericarp colorant, elderberry colorant, caramel, gardenia colorant, corn colorant, saffron colorant and carotene; preservatives such as pectin decomposition products, benzoic acid, sorbic acid, parabens and potassium sorbate; thickeners such as sodium alginate, propylene glycol alginate, calcium cellulose glycolate and sodium cellulose glycolate; antioxidants such as L-ascorbic acid, tocopherol, erythrobic acid and rutin; color developing agents such as ferrous sulfate, sodium nitrite and potassium nitrate; bleaching agents such as sodium hydrogen nitrite and potassium metabisulfite; quality keeping agents such as propylene glycol; quality improving agents such as L-cysteine hydrochloride and calcium stearyl lactate; inflating agents such as ammonium chloride, potassium hydrogen D-tartrate, ammonium carbonate, potassium carbonate, sodium hydrogen carbonate and alum; emulsifiers such as lecithin, sphingo-lipids, vegetable sterols, soybean saponin, sodium alginate, propylene glycol alginate, casein sodium, glycerol fatty acid esters, sucrose fatty acid esters and sorbitan fatty acid esters; emulsion stabilizers such as sodium chondroitin sulfate; flavoring substances such as lemon oil, eucalyptus oil, peppermint oil, vanilla extract, orange oil, garlic oil, ethyl acetoacetate, anisaldehyde, ethyl vanillin, cinnamic acid, citronellyl acetate, citral, vanillin, butyl butyrate and esters; nourishing agents such as L-ascorbic acid, L-asparagine, L-alanine, inositol, L-glutamine, carotene, tocopherol, vitamin A, folic acid, iron citrate, heme iron and uncalcined calcium; wheat flour-improving agents such as benzoyl peroxide, ammonium persulfate and chlorine dioxide; bactericides such as bleaching powder, hydrogen peroxide and hypochlorous acid; chewing gum bases such as methyl acetylricinolate, ester gum, vinyl acetate resin, polyisobutylene and polybutene; anti-blocking agents such as D-mannitol; integrating agents such as acidic sodium pyrophosphate, potassium pyrophosphate and sodium pyrophosphate; acidifiers such as adipic acid, citric acid, gluconic acid, succinic acid, D-tartaric acid, lactic acid and DL-malic acid; and seasonings such as fish extract, yeast extract, sea tangle extract, soy sauce, tomato puree, meat extract, *mirin* (sweetened sake for seasoning), fruit puree, dried bonito, sodium L-aspartate, DL-alanine, L-arginine L-glutamate, disodium 5'-inosinate, trisodium citrate, L-glutamic acid, sodium L-glutamate, succinic acid, L-tartaric acid and sodium lactate.

The oolong tea extract provided by the present invention can be administered as a skin external preparation either as such (i.e., as the extract) or after diluting with water, etc. or concentrating. Alternatively the oolong tea extract may be optionally diluted with water, concentrated or processed into a powder or granules and then formulated together with publicly known pharmaceutical carriers into aerosols, solutions, thickened extracts, suspensions, emulsions, ointments, cataplasmas, liniments and lotions. Alternatively, the oolong tea extract may be blended with aqueous components, surfactants, oily components, solubilizers, humectants, powdery components, alcohols, pH regulating agents, preservatives, antioxidants, thickeners, colorants, pigments, flavoring substances, etc. appropriately selected from among publicly known ones employed in cosmetics, quasi drugs and drugs. The skin external preparations may be in the dosage form of lotions, gels, emulsions, ointments, etc. Also, it may be provided in the form of various cosmetics such as lotions (softening lotions, astringent lotions, etc.), creams (emollient creams, moisture creams, massage creams, etc.), milky lotions (emollient milky lotions, nourishing milky lotions, cleansing milky lotions, etc.), face cleansers, skin cleansers, makeup cosmetics (foundations, eye-colors, cheek-colors, lipsticks, etc.), hair care products (shampoos, rinses, hair-treatments, hair creams, hair tonics, hair nourishments, hair growth stimulants, etc.) or bathing compositions (bath oils, bath salts, foam bath, etc.).

When the oolong tea extract of the present invention is to be used as an agent for preventing or relieving inflammation or allergy, the dose of the oolong tea extract (in terms of the solid matters) according to the present invention may widely vary depending on the severity of the symptoms, relative health status, age, sex, body weight, etc. of the subject to whom it is administered. Usually, the effect can be achieved by administering the oolong tea extract in a dose of at least 0.01 g/kg per day. The daily dose of the oolong tea extract may range from 0.6 to 20 g (in terms of the solid matters) in the case of a usual male adult weighing 60 kg. It may be administered once to several ten times per day. A daily dose thereof exceeding 20 g causes neither any problem in safety nor disorders. When a commercially available oolong tea beverage is used as such as the oolong tea extract, an efficacious daily dose of 0.6 g/day of the oolong tea extract (in terms of the solid matters) in the case of a usual male adult weighing 60 kg seemingly corresponds to 200 to 300 ml of the oolong tea beverage.

### EXAMPLES

To further illustrate the present invention in greater detail, and not by way of limitation, the following Test Example and Examples will be given.

### Example 1

### Production of oolong tea extract:

To 100 g of oolong tea leaves was added 1,000 ml of deionized water. After extracting at 40°C for 30 minutes, the mixture was filtered and the filtrate thus obtained was centrifuged. Then the obtained supernatant was pasteurized at 95°C for 6 seconds and freeze-dried to thereby give 28 g of an extract.

### Test Example 1

### Inhibitory effect on mouse ear contact dermatitis:

ICR mice (7-weeks-old) were purchased from Clea Japan and preliminarily fed for 1 week before testing. Feeding was performed in a feeding room under regulated conditions [room temperature: 23 ± 1 to 2°C, humidity: 55 ± 5%, ventilation rate: 12 to 15 times/hr (the all fresh air system), illumination: 12 hours/day (from 7:00 a.m. to 7:00 p.m.)] by using polyisopentene cages [manufactured by Japan Charles River, 235 x 325 x 170 (height) mm] each having 6 animals. The animals were allowed to take a solid feed CE-2 (Manufactured by Clea Japan) and drinking water *ad libitum*.

The oolong tea extract obtained in Example 1 was dissolved in distilled water to thereby prepare samples giving 3 doses of 0.01, 0.03 and 0.1 g/ 10 ml/kg.

The mice were classified into groups each having 10 animals. 100 µl of a 1.5% solution of 2,4-dinitrofluorobenzene (DNFB) in ethanol was subcutaneously injected into the back of each mouse and sensitization was thus established in 5 days. On the day 6, a 1% solution of DNFB in olive oil was applied to the right ear to thereby induce ear contact dermatitis. 24 hours thereafter, both ears were holed with a punch of 8 mm in diameter and weighed. The oolong tea extract was orally administered from the day before the initial sensitization to the day of the edema-induction, i.e., continuously for 7 days. To a control group, distilled water was administered. The results of the test were expressed in mean ± standard error and significant differences among groups were examined by Student's t-test. Fig. 1 shows the results.

As Fig. 1 indicates, DNFB clearly caused an increase in ear weight in the control group with the administration of distilled water, while the increase in ear weight due to DNFB was remarkably suppressed in each of the test groups with the administration of 0.01, 0.03 and 0.1 g/kg of the oolong tea extract.

### Test Example 2

### Inhibitory effect on mouse delayed-type foot edema:

ICR mice (8-weeks-old) were purchased from Clea Japan and preliminarily fed for 1 week before testing. Feeding was performed in a feeding room under regulated conditions [room temperature: 23 ± 1 to 2°C, humidity: 55 ± 5% ventilation rate: 12 to 15 times/hr (the all fresh air system), illumination: 12 hours/day (from 7:00 a.m. to 7:00 p.m.)] by using polyisopentene cages [manufactured by Japan Charles River, 235 x 325 x 170 (height) mm] each having 6 animals. The animals were allowed to take a solid feed CE-2 (Manufactured by Clea Japan) and drinking water *ad libitum*.

The oolong tea extract obtained in Example 1 was dissolved in distilled water to thereby prepare samples giving 3 doses of 0.01, 0.03 and 0.1 g/10 ml/kg.

The mice were classified into groups each having 10 animals. Each animal was sensitized by subcutaneously injecting 10⁷/25 µl sheep red blood cells (SRBC) prepared from preserved sheep blood into the right hind footpad. 4 days thereafter, 10⁸/25 µl of SRBCs were subcutaneously injected into the left hind footpad to thereby induce delayed-type foot edema. After 24 hours, the degree of the edema was measured by using a sickness gauge and the difference in edema between the right and left feet was evaluated as the edema ratio. The oolong tea extract was orally administered since the sensitization continuously for 4 days. To a control group, distilled water was administered. The results of the test were expressed in mean ± standard error and significant differences among groups were examined by Student's t-test. Fig. 2 shows the results.

As Fig. 2 indicates, SRBC clearly induced foot edema in the control group with the administration of distilled water, while the foot edema due to SRBC was remarkably suppressed in each of the test groups with the administration of 0.01, 0.03 and 0.1 g/kg of the oolong tea extract.

### Test Example 3

### Inhibitory effect on mouse ear contact dermatitis:

ICR mice (7-weeks-old) were purchased from Clea Japan and preliminarily fed for 1 week before testing. Feeding was performed in a feeding room under regulated conditions [room temperature: 23 ± 1 to 2°C humidity: 55 ± 5%, ventilation rate: 12 to 15 times/hr (the all fresh air system), illumination: 12 hours/day (from 7:00 a.m. to 7:00 p.m.)] by using polyisopentene cages [manufactured by Japan Charles River, 235 x 325 x 170 (height) mm] each having 6 animals. The animals were allowed to take a solid feed CE-2 (Manufactured by Clea Japan) and drinking water *ad libitum*.

The oolong tea extract obtained in Example 1 was dissolved in distilled water to thereby prepare a sample giving a dose of 0.5 g/10 ml/kg.

The mice were classified into groups each having 10 animals. 100 µl of a 1.5% solution of 2,4-dinitrofluorobenzene (DNFB) in ethanol was subcutaneously injected into the back of each mouse and sensitization was thus established in 5 days. On the day 6, a 1% solution of DNFB in olive oil was applied to the right ear to thereby induce ear contact dermatitis. 24 hours thereafter, segments obtained from both ear lobes were subjected to HE staining and Giemsa staining and the otic capsules were histologically observed to thereby measure the epidermic cell count (epidermis thickness), corium thickness (x 1/40 mm), nucleated cell count and mast cell count per unit area (1/4² mm²). The oolong tea extract was orally administered from the day before the initial sensitization to the day of the edema-induction, i.e., continuously for 7 days. To a control group, distilled water was administered. The results of the test were expressed in mean ± standard error and significant differences among groups were examined by Student's t-test. Table 1 shows the results.

**[Table 1]**

| Data | Test group (oolong tea extract) | | Control group (distilled water) | |
|---|---|---|---|---|
| | Right | Left | Right | Left |
| Epidermis thickness mean | 2.16 | 1.66 | 2.34 | 1.28 |
| standard error | 0.07180 | 0.11944 | 0.12667 | 0.08537 |
| Corium thickness mean | 8.13** | 6.23 | 10.51 | 5.64 |
| standard error | 0.72358 | 0.39470 | 1.08017 | 0.49915 |
| Nucleated cell count mean | 74.38* | 58.84 | 98.98 | 49.74 |
| standard error | 5.21787 | 3.90291 | 10.41522 | 2.53457 |
| Mast cell count mean | 8.20** | 7.86 | 4.52 | 7.54 |
| standard error | 0.54406 | 0.66403 | 0.23702 | 0.80777 |

| | | | | |
|---|---|---|---|---|
| *, ** and ***: P <0.05, P < 0.01 and P < 0.001 vs. the control group. | | | | |

As Table 1 indicates, the test group with the administration of the oolong tea extract showed a small number of monocytes infiltrating the corium in the ear part due to DNFB-induction and a significantly large number of mast cells compared with the control group. Thus, it is suggested that the oolong tea extract acts on both of monocytes and mast cells so as to inhibit allergic contact dermatitis.

### Example 2

### Production of oolong tea extract:

29.5 g of oolong tea leaves made in China were extracted 16 times as much deionized water at 90°C for 3 minutes and 30 seconds. After filtering, an extract was obtained. Then deionized water was added thereto so as to give a final volume of 1,000 ml. In order to relieve the bitterness at taking, 3.5 g/l of sugar was added. Further, ascorbic acid and sodium hydrogencarbonate were added to thereby adjust the pH value of the extract to 5.8. This extract had a concentration (Brix) of 0.55%. Next, the extract was packed into cans of 190 g in capacity and retorted at 124°C for 8 minutes to thereby give an oolong tea beverage packed in 190 ml cans.

### Example 4

### Relieving effect on patient with atopic dermatitis:

The subjects employed were outpatients and inpatients (48 in total) with diagnosis as atopic dermatitis who had explanation about this test and consented to participate therein. Under doctors' administration, the oolong tea extract (oolong tea beverage) obtained in Example 2 was orally administered to the patients in a daily dose of 380 ml (i.e., two 190 ml cans) continuously for 4 weeks. The subjects were allowed to take the oolong tea extract without restriction in time, frequency and a single dose except the daily dose. Evaluation was made thrice (i.e., the day of the initiation of the test and two and four weeks thereafter) on 5 items (itching, lichenification, papule, erythema and skin dryness). At the termination of the test, changes in these symptoms were evaluated in total and the efficacy was judged in 4 grades (highly efficacious, efficacious, not efficacious, worsened). Side-effects occurring during the test period were put on record (symptom, appearance date, disappearance date, degree, relation to oolong tea extract, treatment with oolong tea extract, consequence and remarks by doctor in charge). After the completion of the test, the safety was judged in 4 grades (no side-effect, slight side-effect, moderate side-effect, serious side-effect) by taking the type and degree of the side-effects observed during the administration period into consideration. Moreover, the usefulness was judged in 4 grades (highly useful, useful, hard to judge, undesirable) by totally taking the efficacy and safety into consideration.

Table 2 shows the background factors of the patients of 48 cases. The subjects included 27 males and 21 females aged from 6 to 47 years (22 years on average). Regarding the severity, eight severe cases, 37 moderate cases and three slight cases were included therein.

**[Table 2]**

| No. of cases | | 48 in total |
|---|---|---|
| Sex | male | 27 |
| | female | 21 |
| Age | 6 - 10 | 2 |
| | 11 - 20 | 20 |
| | 21 - 30 | 17 |
| | 31 - 40 | 8 |
| | 41 - 50 | 1 |
| Severity | serious | 8 |
| | moderate | 37 |
| | slight | 3 |

Table 3 shows the results of the evaluation of efficacy including 12 (25%) highly efficacious cases, 19 (40%) efficacious cases, 15 (31%) not efficacious cases and 2 (4%) worsened cases, thus showing the ratio of the cases with efficacy of 65%.

**[Table 3]**

| Efficacy | Highly efficacious | Efficacious | Not efficacious | Worsened | Total |
|---|---|---|---|---|---|
| Case (no.) | 12 | 19 | 15 | 2 | 48 |
| Case (%) | 25 | 40 | 31 | 4 | 100 |
| Accumulative (%) | 65 | | 35 | | 100 |

Regarding side-effects, one patient among 48 complained of heavy load on the stomach. However, the administration was continued, since the symptom was a slight one and the patient didn't wish to stop the administration. With respect to safety, a slight side-effect was observed in one case while none was observed in 47 cases. Neither moderate nor serious side-effect was observed in any case. The ratio of safety amounted to 98%.

Table 4 shows the results of the evaluation on usefulness including 12 (25%) highly useful cases, 18 (38%) useful cases, 16 (33%) cases hard to judge and 2 (4%) undesirable cases, thus showing the ratio of usefulness of 63%.

**[Table 4]**

| Usefulness | Highly useful | Useful | Hard to judge | Undesirable | Total |
|---|---|---|---|---|---|
| Case (no.) | 12 | 18 | 16 | 2 | 48 |
| Case (%) | 25 | 38 | 33 | 4 | 100 |
| Accumulative (%) | 63 | | 37 | | 100 |

Based on these results, it has been confirmed that the oolong tea extract is a substance having a high safety and being highly useful as a remedy for atopic dermatitis.

### Example 3:

### Production of tablet:

150 g of the oolong tea extract obtained in Example 1 was mixed with the same amount of lactose and 5 g of magnesium stearate. The obtained mixture was processed into tablets (10 mm in diameter, 300 mg) with the use of a single-tabletting machine.

### Example 4:

### Production of granules:

The tablets obtained in Example 3 were ground, dressed and sieved to thereby give granules of 20 - 50 mesh.

### Example 5:

### Production of candy:

By using the following materials, candies were produced in a conventional manner.

| | |
|---|---|
| powdery sorbitol | 99.7 g |
| flavoring substance | 0.2 g |
| oolong tea extract (produced in Example 1) | 0.05 g |
| sorbitol seed | 0.05 g |
| total | 100 g. |

### Example 6:

### Production of chewing gum:

By using the following materials, chewing gum was produced in a conventional manner.

| | |
|---|---|
| gum base | 10 g |
| calcium carbonate | 2 g |
| stevioside | 0.1 g |
| oolong tea extract (produced in Example 1) | 0.05 g |
| lactose | 76.85 g |
| flavoring substance | 1 g |
| total | 100 g. |

### Example 7:

### Production of mikan (Satsuma mandarin orange) juice:

By using the following materials, *mikan* juice was produced in a conventional manner.

| | |
|---|---|
| frozen concentrated *mikan* juice | 5 g |
| fructose/glucose liquid sugar | 10 g |
| citric acid | 0.2 g |
| L-ascorbic acid | 0.02 g |
| oolong tea extract (produced in Example 1) | 0.05 g |
| flavoring substance | 0.2 g |
| colorant | 0.1 g |
| water | the balance |
| total | 100 g. |

### Example 8:

### Production of oolong tea extract (extract):

100 g of oolong tea leaves were poured into 5,000 ml of boiling purified water. After saturating the tea leaves with the water by stirring well, the mixture was maintained for 5 minutes at 90°C or above. Then the tea leaves were separated from the extract by filtering through a cotton flannel filter to thereby give the oolong tea extract (extract).

### Example 9:

### Production of oolong tea extract (processed extract):

500 g of oolong tea leaves were poured into 5,000 ml of a boiling solution prepared by mixing purified water with ethanol at a weight ratio of 2 : 1. After saturating the tea leaves with the solution by stirring well, the mixture was maintained for 30 minutes at 90°C or above. Then the tea leaves were separated from the extract by filtering through a 100-mesh screen. Further, the filtrate was centrifuged at 3,000 rpm to thereby give 4,000 ml of an oolong tea extract. The obtained extract was kept overnight at 5 °C or below to thereby separate the supernatant from the precipitate. The concentration of the soluble solid matters in the supernatant was regulated to 15% (Brix) and then it was frozen at - 40°C followed by freeze-drying. Thus an oolong tea extract powder was obtained.

### Example 10:

### Production of oolong tea extract beverage:

100 g of oolong tea leaves were poured into 1,000 ml of boiling purified water. After saturating the tea leaves with the water by stirring well, the mixture was maintained for 5 minutes at 90°C or above. Then the tea leaves were separated from the extract by filtering successively through a 100-mesh wire screen and a cotton flannel filter to thereby give the oolong tea extract. The extract was cooled to 30°C and then a small amount of ascorbic acid was added thereto. Then the mixture was heated to 90°C and, in the hot state, packed into cans. After sealing and retorting at 120°C for 15 minutes, it was cooled to room temperatures to thereby give an oolong tea beverage.

### Example 11:

### Production of jelly containing oolong tea extract:

The following materials were employed for the production:

| | |
|---|---|
| oolong tea extract (produced in Example 8) | 2 g |
| sugar | 500 g |
| starch syrup | 500 g |
| pectin | 13 g |
| citric acid | 4 g |
| sodium citrate | 1.5 g |
| flavoring substance | 1 cc |
| edible colorant | 0.2 g |

13 g of pectin was mixed with 20 g of sugar and then the obtained mixture was dissolved in 330 cc of water with caution so as not to form undissolved portions. Further, citric acid and sodium citrate were added thereto and the resulting mixture was boiled. After adding starch syrup, the mixture was heated to 100°C. Then the residual sugar was added thereto and the mixture was heated to 109°C. After allowing to stand for several minutes, the oolong tea extract, flavoring substance and colorant were added. The obtained mixture was stirred and filled into a starch mold. Then it was dried at 50°C or below for 10 hours or longer and thus a jelly containing the oolong tea extract was obtained.

### Example 12

### Production of cocky containing oolong tea extract:

The following materials were employed for the production:

| | |
|---|---|
| oolong tea extract (produced in Example 8) | 2 g |
| sugar | 430 g |
| weak wheat flour | 680 g |
| butter (salt-free) | 220 g |
| whole egg | 150 g |
| baking powder | 6 g |

Butter was softened and sugar was added thereto. The obtained mixture was vigorously stirred until it became creamy. Then whole egg was added thereto and the mixture was further stirred. Next, weak wheat flour and baking powder were added thereto and the mixture was lightly stirred crosswise. Finally, the oolong tea extract was added thereto. The mixture was squeezed out of a pastry bag onto an oven plate. After molding, it was baked in a pre-heated oven at 180°C for 11 to 13 minutes to thereby give cookies containing the oolong tea extract.

### Example 13:

### Production of kamaboko containing oolong tea extract:

The following materials were employed for the production:

| | |
|---|---|
| oolong tea extract (produced in Example 8) | 1 g |
| ground Alaska pollack meat | 100 g |
| sodium chloride | 20 g |
| seasoning | 2 g |
| egg albumen | 10 g |

Sodium chloride, seasoning and the oolong tea extract were added to the ground Alaska pollack meat. After kneading and maturing, the mixture was molded and steamed in a steamer pre-heated to about 90 to 95°C. Then it was cooled by allowing to stand to thereby give a *kamaboko* containing the oolong tea extract.

### Example 14

### Production of emollient cream containing oolong tea extract:

By using the following materials, an emollient cream was produced in a conventional manner:

| | |
|---|---|
| beeswax | 2.0 g |
| stearyl alcohol | 5.0 g |
| stearic acid | 8.0 g |
| squalane | 10.0 g |
| self-emulsified propylene glycol monostearate | 3.0 g |
| polyoxyethylene cetyl ether | 1.0 g |
| perfume | 0.5 g |
| preservative | q.s. |
| antioxidant | q.s. |
| propylene glycol | 7.8 g |
| glycerin | 4.0 g |
| sodium hyaluronate | 0.1 g |
| oolong tea extract (produced in Example 1) | 0.1 g |
| triethanolamine | 1.0 g |
| purified water | 57.5 g |

### Example 15

### Production of shampoo containing oolong tea extract:

By using the following materials, a shampoo was produced in a conventional manner:

| | |
|---|---|
| alkyl ether sodium sulfate | 16.0 g |
| diethanolamide laurate | 4.0 g |
| propylene glycol | 1.9 g |
| oolong tea extract (produced in Example 1) | 0.1 g |
| preservative, colorant, perfume | q.s. |
| purified water | 78.0 g |

The antiallergic agent, antiinflammatory agent, anti-atopic dermatitis agent or antipsoriatic agent of the present invention is efficacious in preventing, reducing or relieving symptoms due to inflammation or allergic reactions. In particular, it is useful in preventing, reducing or relieving the symptoms of atopic dermatitis and psoriasis. Because of containing oolong tea extract as the active ingredient, the agent of the present invention is excellent in safety and exerts no side-effect even over continuous and prolonged administration. Further, the everyday intake of the foods or beverages of the present invention containing oolong tea extract contributes to the prevention of symptoms due to allergic reactions. Also, inflammation and swollen throat in association with cold, pollen hypersensitivity, coughing, etc. can be prevented or relieved thereby. The antiallergic agent, antiinflammatory agent, anti-atopic dermatitis agent or antipsoriatic agent of the present invention can be employed also in the form of a skin external preparation which is efficacious in reducing or relieving inflammation. When contained in cosmetics, furthermore, it can be used every day.

### Drawings

### [Fig. 1]

a Fig. 1 Effect of oolong tea extract on DNFB-induced mouse ear contact dermatitis
b * and **: P < 0.05 and P < 0.01 vs. control group
c ear edema weight (mg)
d distilled water
e oolong tea extract (g/kg/day x 7 days)

### [Fig. 2]

a Fig. 2 Effect of oolong tea extract on SRBC-induced mouse delayed-type foot edema
b * and **: P < 0.05 and P < 0.01 vs. control group
c foot edema (mm)
d distilled water
e oolong tea extract (g/kg/day x 4 days)

## Claims

1. An antiallergic agent characterized by containing oolong tea extract as the active ingredient.

2. An antiinflammatory agent characterized by containing oolong tea extract as the active ingredient.

3. An antiallergic agent or an antiinflammatory agent characterized by containing oolong tea extract as the active ingredient as claimed in Claim 1 or 2 which aims at preventing, reducing or relieving atopic dermatitis.

4. An antiallergic agent or an antiinflammatory agent characterized by containing oolong tea extract as the active ingredient as claimed in Claim 1 or 2 which aims at preventing, reducing or relieving psoriasis.

5. An oral antiallergic agent or an oral antiinflammatory agent which is an antiallergic agent or an antiinflammatory agent characterized by containing oolong tea extract as the active ingredient as claimed in anyone of Claims 1 to 4 to be administered orally.

6. Foods or beverages which contain an oral antiallergic agent or an oral antiinflammatory agent characterized by containing oolong tea extract as the active ingredient as claimed in Claim 5.

7. Use of an oral antiallergic agent or an oral antiinflammatory agent as claimed in Claim 5 in the production of foods or beverages in order to prevent, reduce or relieve allergic symptoms or inflammatory symptoms.

8. An external antiallergic agent or an external antiinflammatory agent which is an antiallergic agent or an antiinflammatory agent characterized by containing oolong tea extract as the active ingredient as claimed in anyone of Claims 1 to 4 in the form of a skin external preparation.

9. Cosmetics containing an external antiallergic agent or an external antiinflammatory agent characterized by containing oolong tea extract as the active ingredient as claimed in Claim 8.

10. An anti-atopic dermatitis agent characterized by containing oolong tea extract as the active ingredient.

11. An antipsoriatic agent characterized by containing oolong tea extract as the active ingredient.

12. An oral anti-atopic dermatitis agent or an oral antipsoriatic agent which is an anti-atopic dermatitis agent or an antipsoriatic agent characterized by containing oolong tea extract as the active ingredient as claimed in Claim 10 or 11 to be administered orally.

13. Foods or beverages which contain an oral anti-atopic dermatitis agent or an oral antipsoriatic agent characterized by containing oolong tea extract as the active ingredient as claimed in Claim 12.

14. Use of an anti-atopic dermatitis agent or an oral antipsoriatic agent as claimed in Claim 12 in the production of foods or beverages in order to prevent, reduce or relieve atopic dermatitis or psoriasis.

15. An external anti-atopic dermatitis agent or an external antipsoriatic agent which is an anti-atopic dermatitis agent or an antipsoriatic agent characterized by containing oolong tea extract as the active ingredient as claimed in Claim 10 or 11 in the form of a skin external preparation.

16. Cosmetics containing an external anti-atopic dermatitis agent or an external antipsoriatic agent characterized by containing oolong tea extract as the active ingredient as claimed in Claim 15.
